(19) Europäisches Patentamt
European Patent Office
Office européen des brevets

(11) EP 2 752 187 A1

(12) # EUROPEAN PATENT APPLICATION
published in accordance with Art. 153(4) EPC

(43) Date of publication:
**09.07.2014 Bulletin 2014/28**

(21) Application number: **12827149.1**

(22) Date of filing: **08.08.2012**

(51) Int Cl.:
***A61K 8/55*** *(2006.01)* ***A61K 8/34*** *(2006.01)*
***A61K 8/37*** *(2006.01)* ***A61K 8/86*** *(2006.01)*
***A61K 8/92*** *(2006.01)* ***A61Q 5/06*** *(2006.01)*

(86) International application number:
**PCT/JP2012/070173**

(87) International publication number:
**WO 2013/031496 (07.03.2013 Gazette 2013/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2011 JP 2011190764**

(71) Applicants:
- **Daicel Corporation**
  **Osaka-shi, Osaka 530-0001 (JP)**
- **Nihon University**
  **Tokyo 102-8275 (JP)**

(72) Inventors:
- **SAKANISHI, Yuichi**
  **Ohtake-shi**
  **Hiroshima 739-0695 (JP)**
- **HASHIZAKI, Kaname**
  **Tokyo 102-8275 (JP)**
- **SAITO, Yoshihiro**
  **Tokyo 102-8275 (JP)**
- **TAGUCHI, Hiroyuki**
  **Tokyo 102-8275 (JP)**

(74) Representative: **Grünecker, Kinkeldey, Stockmair & Schwanhäusser**
**Leopoldstrasse 4**
**80802 München (DE)**

(54) **HAIR OIL USING OIL-BASED GEL COMPOSITION**

(57) Provided is a hair oil that spreads smoothly, gives a soft and light finish, and satisfactorily effectively imparts a moist feel, easiness of styling, and a glossy feel to the hair. The provided hair oil includes an oil-based gel-like composition including a gel-forming agent and an oil-phase component,
in which:
the gel-forming agent includes a lecithin; and at least one selected from a polyglycerol and a polyglycerol fatty acid ester in an amount (total amount) of from 5 to 100 percent by weight per 100 percent by weight of the lecithin;
the polyglycerol has a degree of polymerization of from 2 to 20; and
the polyglycerol fatty acid ester has a fatty acid residue having 14 or less carbon atoms, has an HLB of 15 or more as calculated based on an organic conceptual diagram, and has a degree of polymerization of from 8 to 40.

EP 2 752 187 A1

## Description

Technical Field

The present invention generally relates to hair oils using oil-based gel-like compositions. More specifically, the present invention relates to a hair oil including an oily component in combination with a gel-forming agent including a lecithin, a specific polyglycerol or/and a specific polyglycerol fatty acid ester, which hair oil spreads smoothly during use, gives a satisfactorily soft and light finish, and highly effectively imparts a moist feel, easiness of styling, and a glossy feel to the hair.

Background Art

Hair oils are a kind of hair cosmetics and used so as to supplement oily components to the hair to give luster, smoothness, flexibility, and other properties to the hair. The hair oils have employed a vegetable oil such as camellia oil or olive oil; or a mineral oil such as liquid paraffin as a principal component. Such principal component has been incorporated typically with a higher fatty acid and a silicone oil so as to regulate the feeling of use (see, for example, Non Patent Literature (NPL) 1). In particular, various types of silicone oils having different structures and/or different molecular weights have been used to give smoothness and softness during and after use and to provide a light finish. To provide further better feeling of use, the combination use of a high-molecular-weight silicone typically with a low-molecular-weight silicone or volatile silicone has been employed (see, for example, Patent Literature (PTL) 1 and 2).

Citation List

Patent Literature

PTL 1: Japanese Unexamined Patent Application Publication (JP-A) No. S63-183517
PTL 2: JP-A No. H01-175923

NPL 1: "Shin Keshohin-gaku (in Japanese; New Cosmetology) written by Takeo Mitsui (First Edition; published on January 12, 1993, Nanzando Co., Ltd.) pp. 436-439

Summary of Invention

Technical Problem

These oil compositions, however, generally have low viscosities and thereby disadvantageously fall down from between fingers or are applied to an unintended portion of the hair upon application to the hair. In turn, an oily component (oil composition), when prepared using an already-existing gelling agent, very poorly spreads upon application.
Accordingly, an object of the present invention is to provide a hair oil that spreads smoothly during use and imparts a soft and light finish, a moist feel, easiness of styling, and a glossy feel to the hair.

Solution to Problem

Under these circumstances, the present inventors made intensive investigations to allow a hair oil to have better usability and to exhibit an additional function or functions. As a result, they have found a hair oil including an oily component in combination with a gel-forming agent containing specific amounts of a lecithin and at least one of a specific polyglycerol and a specific polyglycerol fatty acid ester; and have found that this hair oil can spread smoothly during use, gives a satisfactorily soft and light finish, and highly effectively imparts a moist feel, easiness of styling, and a glossy feel to the hair. The present invention has been made based on these findings.
Specifically, the present invention provides:

a hair oil including an oil-based gel-like composition, the composition including: a gel-forming agent; and an oil-phase component,

in which:

the gel-forming agent includes: a lecithin; and at least one selected from the group consisting of a polyglycerol and a polyglycerol fatty acid ester in a total amount of from 5 to 100 percent by weight per 100 percent by weight of the lecithin;
the polyglycerol has a degree of polymerization of from 2 to 20; and
the polyglycerol fatty acid ester has a fatty acid residue having 14 or less carbon atoms, has an HLB of 15 or more as calculated based on an organic conceptual diagram, and has a degree of polymerization of from 8 to 40.

The oil-based gel-like composition preferably contains 1 to 30 percent by weight of the gel-forming agent; and 70 to 99 percent by weight of the oil-phase component. Advantageous Effects of Invention
The hair oil according to the present invention can spread smoothly during use and can very effectively impart a soft and light finish, a moist feel, easiness of styling, and a glossy feel to the hair.

Description of Embodiments

Oil-based Gel-like Composition

As used herein the term "oil-based gel-like composition" refers to a gel-like composition containing substantially no or a trace amount (e.g., 1 percent by weight or less, preferably 0.2 percent by weight or less) of water. The oil-based gel-like composition is not limited, as long as including a gel-forming agent and an oil-phase component.
The hair oil contains the oil-based gel-like composition in a content of preferably from 1 to 30 percent by weight, more preferably from 3 to 20 percent by weight, and furthermore preferably from 3 to 10 percent by weight based on the total amount of the hair oil. The oil-based gel-like composition, if contained in an excessively low content, may cause insufficient gel formation and fail to give a hair oil in a stable gel state. In contrast, the oil-based gel-like composition, if contained in an excessively high content, may exhibit saturated gel formation and moisturizing/water-retention effects, and such large amount use may give no advantages and be uneconomical. To prevent these, the oil-based gel-like composition is preferably used in a content within the above-specified range.

Gel-forming Agent

The gel-forming agent in the hair oil according to the present invention contains a lecithin; and at least one selected from the group consisting of a polyglycerol and a polyglycerol fatty acid ester in a total content (total amount) of from 5 to 100 percent by weight, preferably from 5 to 80 percent by weight, and more preferably from 10 to 50 percent by weight, per 100 percent by weight of the lecithin. The gel-forming agent, if containing the polyglycerol and/or polyglycerol fatty acid ester in an excessively low total content, may exhibit significantly inferior gel-forming ability, thus being undesirable. In contrast, the gel-forming agent, if containing the polyglycerol and/or polyglycerol fatty acid ester in an excessively high total content, may cause the resulting composition to have a cloudy formulation having a significantly low viscosity, because the polyglycerol derivative itself fails to be solubilized. To prevent these, the gel-forming agent preferably contains the polyglycerol and/or polyglycerol fatty acid ester in a content within the above-specified range.
As will be mentioned below, the lecithin is preferably one containing phosphatidylcholine in a content of from 55 to 99 percent by weight. The gel-forming agent therefore contains at least one selected from the group consisting of a polyglycerol and a polyglycerol fatty acid ester in a content (total amount) of preferably from 5 to 100 percent by weight, more preferably from 5 to 80 percent by weight, and furthermore preferably from 10 to 50 percent by weight, as with above, per 55 to 99 percent by weight (e.g., 75 percent by weight) of phosphatidylcholine.
The gel-forming agent is contained in a content of preferably from 1 to 30 percent by weight, more preferably from 5 to 20 percent by weight, and furthermore preferably from 10 to 15 percent by weight, based on the total amount of the hair oil. The gel-forming agent, if contained in an excessively low content, may cause insufficient gel formation and fail to give a hair oil in a stable gel state. In contrast, the gel-forming agent, if contained in an excessively high content, may exhibit saturated gel formation and moisturizing/water-retention effects, and such large amount use may give no advantages and be uneconomical. To prevent these, the gel-forming agent is preferably contained in a content within the above-specified range.
The lecithin is contained in a content of preferably from 1 to 20 percent by weight, and particularly preferably from 5 to 10 percent by weight, based on the total amount of the hair oil. The lecithin, if contained in an excessively low content, may cause insufficient gel formation and fail to give a hair oil in a stable gel state. In contrast, the gel-forming agent, if contained in an excessively high content, may exhibit saturated gel formation and moisturizing/water-retention effects, and such large amount use may give no advantages and be uneconomical. To prevent these, the gel-forming agent is preferably contained in a content within the above-specified range.
The at least one selected from the group consisting of a polyglycerol and a polyglycerol fatty acid ester is contained in a content (total amount) of preferably from 0.048 to 15 percent by weight, and more preferably from 0.5 to

15 percent by weight, based on the total amount of the hair oil. The at least one selected from the group consisting of a polyglycerol and a polyglycerol fatty acid ester is contained in a content (total amount) of preferably from 5 to 100 percent by weight, and particularly preferably from 5 to 33 percent by weight, based on the total amount of the gel-forming agent. The at least one component, if contained in an excessively low content, may cause insufficient gel formation and fail to give a hair oil in a stable gel state. In contrast, the at least one component, if contained in an excessively high content, may exhibit saturated gel formation and moisturizing/water-retention effects, and such large amount use may give no advantages and be uneconomical. To prevent these, the at least one component is preferably contained in a content within the above-specified range.

Oil-phase Component

The oil-phase component for use in the present invention may contain, as a principal component, a polar oil alone or in combination with a nonpolar oil as a mixture, or a nonpolar oil alone. The nonpolar oil is exemplified by hydrocarbons such as squalane, petrolatum, and liquid paraffin; and chain or cyclic silicone oils. The polar oil is exemplified by oils and fats such as olive oil; waxes such as lanolin; esters [e.g., esters of fatty acids having 8 or more carbon atoms (preferably 8 to 25 carbon atoms) with alcohols], such as isopropyl myristate, decyl oleate, cetyl 2-ethylhexanoate, and glyceryl tri-2-ethylhexanoate; higher fatty acids [e.g., fatty acids having 12 or more carbon atoms (preferably 12 to 25 carbon atoms)], such as oleic acid and lauric acid; and higher alcohols that are solid at room temperature [e.g., alcohols having 12 or more carbon atoms (preferably 12 to 25 carbon atoms)], such as cetanol. Each of these oil-phase components may be used alone or in combination in a content of from 70 to 99 percent by weight based on the total amount of the oil-based gel-like composition. The oil-phase component, if contained in a content (concentration) of less than 70 percent by weight, may cause an excessively large amount of the gel-forming agent; and, if contained in a content of more than 99 percent by weight, may adversely affect the gel stability and cause economical disadvantages. To prevent these, the oil-phase component is preferably contained in a content within the above-specified range. The oil-phase component is contained in a content of preferably from 70 to 99 percent by weight, and more preferably from 80 to 97 percent by weight, based on the total amount of the oil-based gel-like composition.

Polyglycerol Fatty Acid Ester

Polyglycerol fatty acid esters for use in the present invention may be produced by a variety of processes. The processes are exemplified by (1) a process of subjecting glycerol to addition polymerization with glycidol and esterifying the resulting compound with a fatty acid; (2) a process of adding glycidol to a fatty acid; and, relating to this, (3) a process of adding hydroxyl-protected glycidol to glycerol and deprotecting the resulting compound, repeating this procedure up to an arbitrary degree of polymerization, and esterifying the resulting compound with a fatty acid; and (4) a process of thermally condensing glycerol in the presence of a base and esterifying the resulting compound with a fatty acid. Among them, the process (1) is most preferred as the production process, and the resulting polyglycerol fatty acid ester is advantageously usable as the gel-forming agent in the oil-based gel-like composition.

The polyglycerol fatty acid ester for use herein has a fatty acid residue having 14 or less carbon atoms, and more preferably has a fatty acid residue having 6 to 14 carbon atoms. A polyglycerol fatty acid ester having a fatty acid residue having more than 14 carbon atoms may cause the resulting composition to have an emulsion formulation and fail to give a transparent gel. The fatty acid residue may be straight chain or branched chain, but is preferably straight chain. A fatty acid corresponding to the fatty acid residue may be a saturated fatty acid or unsaturated fatty acid, but is preferably a saturated fatty acid. The fatty acid corresponding to the fatty acid residue is specifically exemplified by hexanoic acid, heptanoic acid, octanoic acid, nonanoic acid, decanoic acid, undecanoic acid, dodecanoic acid, and tetradecanoic acid. The polyglycerol fatty acid ester may have one or more of such fatty acid residues. When the polyglycerol fatty acid ester has two or more fatty acid residues, the fatty acid residues may be the same as or different from one another.

The polyglycerol fatty acid ester for use herein has a degree of glycerol unit polymerization of from 8 to 40, and particularly preferably from 8 to 20. A polyglycerol fatty acid ester having a degree of polymerization of less than 8 may fail to give a stable gel. A polyglycerol fatty acid ester having a degree of polymerization of more than 40 may cause the resulting composition to have an emulsion formulation, fail to give a transparent gel, and, in addition, fail to give a gel itself.

Such polyglycerol fatty acid esters for use herein have an HLB of 15 or more, and more preferably from 15 to 20 as calculated based on an organic conceptual diagram. A polyglycerol fatty acid ester having an HLB of less than 15 as calculated based on the organic conceptual diagram may give a transparent solution and fail to allow the resulting composition to have a gel formulation. In contrast, a polyglycerol fatty acid ester having an HLB of from 15 to 20 as calculated based on the organic conceptual diagram may help the resulting composition to have not an emulsion formulation, but a gel formulation more easily. The polyglycerol fatty acid ester preferably has an HLB of 20 or less as calculated based on the organic conceptual diagram so as to help the resulting composition to have not an emulsion

formulation, but a gel formulation more easily. As used herein the term "HLB as calculated based on the organic conceptual diagram" refers to a value determined according to Formula (A) as follows:

$$HLB=\Sigma(\text{Inorganic values})/\Sigma(\text{Organic values})\times 10 \quad (A)$$

(Reference: "Formulation Design with Organic Conceptual Diagram," Nihon Emulsion Co., Ltd.)

Each of different polyglycerol fatty acid esters may be used in combination or alone.

Polyglycerol

Polyglycerols for use in the present invention may be produced by a variety of processes. The processes are exemplified by (1) a process of subjecting glycerol to addition-polymerization with epichlorohydrin, ring-closing the resulting compound through dehydrochlorination under basic conditions, and ring-opening with a diluted sulfuric acid, and repeating this procedure until a target degree of polymerization is obtained; (2) a process of adding glycidol to glycerol; and relating to this, (3) a process of adding hydroxyl-protected glycidol to glycerol, deprotecting the resulting compound, and repeating this procedure until an arbitrary degree of polymerization is obtained; (4) a process of thermally condensing glycerol in the presence of a base; and (5) a process of adding an allyl halide to glycerol and epoxidizing the glycerol, ring-opening the resulting epoxide with water, and repeating this procedure until an arbitrary degree of polymerization is obtained. Among them, the process (2) is most preferred as the production process, and the resulting polyglycerol is advantageously usable as the gel-forming agent in the hair oil.

Polyglycerols for use herein have a degree of polymerization of from 2 to 20. A polyglycerol having a degree of polymerization of less than 2 may fail to give a stable gel; whereas a polyglycerol having a degree of polymerization of more than 20 may cause the resulting composition to have an emulsion formulation, to fail to give a transparent gel and, in addition, to fail to give a gel itself. Polyglycerols having a degree of polymerization of from 2 to 10 are preferably used, of which those having a degree of polymerization of from 3 to 10 are particularly preferred. Each of different polyglycerols having different degrees of polymerization may be used alone or in combination.

Lecithin

The lecithin is a lipid product that includes phosphatidylcholine as a principal component, is widely distributed in natural living bodies such as animals, plants (vegetables), and microorganisms, and is known to be contained in a large amount typically in the liver, yolks, soybeans, and yeasts. Such lecithins are represented by yolk lecithin and soybean lecithin. Each of different lecithins may be used alone or in combination. The lecithin for use herein is preferably one having a phosphatidylcholine content of from about 55 to about 99 percent by weight. A lecithin having a phosphatidylcholine content within this range easily becomes creamy, has a suitable consistency, remains on the skin without falling off therefrom when applied to the skin, and gives comfortable feeling upon use. Natural lecithins are present only in the L-$\alpha$-form, but lecithins of other forms are also usable herein. The natural lecithins are susceptible to oxidation and are unstable. To mitigate this disadvantage, the natural lecithins are preferably hydrogenated upon use according to a known procedure. The term "lecithin(s)" as used herein also includes such hydrogenated lecithins.

The term "phosphatidylcholine" refers to an ester obtained by reacting glycerol (glycerin) with at least one unsaturated fatty acid and phosphoric acid. Protons of the phosphoric acid are substituted with choline acting as an amine functional group. The "phosphatidylcholine" herein also includes a phosphatidylcholine with unsaturated bond(s) being hydrogenated.

The phosphatidylcholine for use herein is particularly specified by General Formula (I) below. In the formula, $R_1$ and $R_2$ each independently represent an aliphatic hydrocarbon group derived from (corresponding to) a saturated or unsaturated fatty acid having 4 to 24 carbon atoms. Specifically, the aliphatic hydrocarbon group is a saturated or unsaturated aliphatic hydrocarbon group having 3 to 23 carbon atoms. Such aliphatic hydrocarbon groups may be straight- or branched-chain and may each substituted with one or more hydroxyl functional groups and/or amine functional groups. X represents a choline residue. The phosphatidylcholine may be any one of the compounds represented by Formula (I), or a mixture of two or more of them. Formula (I) is expressed as follows:

[Chem. 1]

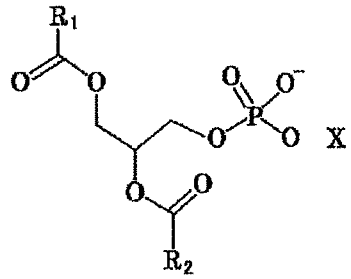

(I)

In one of embodiments of the present invention, fatty acids ($R_1COOH$ and $R_2COOH$) corresponding to $R_1$ and $R_2$ are selected typically from butyric acid, caproic acid, caprylic acid, capric acid, caproleic acid, lauric acid, lauroleic acid, myristic acid, myristoleic acid, palmitic acid, palmitoleic acid, stearic acid, oleic acid, linoleic acid, linolenic acid, arachidic acid, isostearic acid, dihydroxystearic acid, and ricinoleic acid.

A non-hydrogenated phosphatidylcholine (PC) suitable to prepare the composition for use herein can be derived from a "natural" or "synthetic" maternal.

The "natural" phosphatidylcholine (PC) can be obtained through extraction from an animal source or vegetable source such as soybeans, sunflowers, or eggs. Non-hydrogenated phosphatidylcholines derived from a natural material such as soybeans generally include palmitic acid, stearic acid, palmitoleic acid, oleic acid, linoleic acid, linolenic acid, and fatty acids probably having 20 to 22 carbon atoms as fatty acids to esterify the glycerol. Each of them may be used alone or in combination.

Others

In addition to the oil-based gel-like composition, the hair oil according to the present invention may further include one or more components for use in regular hair cosmetics, within qualitative and quantitative ranges not adversely affecting advantageous effects of the present invention. The components may be aqueous components or non-aqueous components, but are preferably non-aqueous components. The components are exemplified by oily components, surfactants, polyhydric alcohols, polyols, chelating agents, microbicides, antioxidants, viscosity modifiers, astringents, antidandruff agents, ultraviolet absorbers, colorants such as dyestuffs and pigments, flavors, cosmetic components (beauty components) such as vitamins, and aerosol propellants.

The hair oil according to the present invention can be formed typically into a liquid, gel, or multilayer form according to a common procedure. The hair oil can also be used typically as a mist or aerosol according to the type of a vessel to be used in combination.

Examples

The present invention will be illustrated in further detail with reference to several examples below. It should be noted, however, that these examples are by no means intended to limit the scope of the invention.

Examples 1 to 9 and Comparative Examples 1 to 6

Hair oils were prepared to have formulations (in weight percent) given in Tables 1 and 2; and these hair oils were subjected to use tests according to evaluation methods below. The results thereof are also indicated in Tables 1 and 2. Numerical values in the tables indicate blending ratios (in weight percent) of respective components. Reagents, preparation methods, and evaluation methods are indicated as follows.

Reagents

Lecithin used herein was PHOSPHLIPON 90G (having a phosphatidylcholine concentration of 95% or more) supplied by H. Holstein GmbH & Co. KG.

Tetraglycerol used herein was PGL 04P supplied by Daicel Corporation.

Decaglycerol used herein was PGL 10PS supplied by Daicel Corporation.

Polyglycerol-20 used herein was PGL 20P supplied by Daicel Corporation.

Polyglycerol-20 monolaurate used herein was obtained by subjecting PGL 20P (supplied by Daicel Corporation) and commercially available lauric acid to dehydration condensation according to a common procedure.

Polyglycerol-20 monomyristate used herein was obtained by subjecting PGL 20P (supplied by Daicel Corporation) and commercially available myristic acid to dehydration condensation according to a common procedure.

Polyglycerol-40 monomyristate used herein was obtained by subjecting PGL XP (supplied by Daicel Corporation) and commercially available myristic acid to dehydration condensation according to a common procedure.

Cetyl octanoate used herein was CEH supplied by KOKYU ALCOHOL KOGYO CO., LTD.
Sunflower oil used herein was Sunflower Oil supplied by Nikko Chemicals Co., Ltd.
Decamethylpentasiloxane used herein was KF-995 supplied by Shin-Etsu Chemical Co., Ltd.
Isododecane used herein was PERMETHYL 99A supplied by Nihon Koken Kogyo Co., Ltd.
Liquid paraffin used herein was MORESCO WHITE P-100 supplied by MORESCO Corporation.

Preparation Method

All materials were uniformly mixed and dissolved and yielded a series of hair oils.

Evaluation Methods

Each of the hair oils obtained in Examples and Comparative Examples was evaluated by methods as follows.

(1) Thickness and Smoothness

The thickness (slight viscosity) and smoothness of the samples were evaluated by a panel of ten sensory-test experts. Specifically, the hair of each panelist was washed with a commercially available shampoo and half dried with a drier. Each sample in an adequate amount was taken and applied smoothly over the hair. Each panelist performed sensory evaluation on a 1-to-5 scale according to following criteria and rated each sample. The criteria are as follows:

Very good: 5
Good: 4
Average: 3
Poor: 2
Very poor: 1

An average of the ratings was further evaluated according to three-level criteria as follows.

Good: Average rating is 3 or more
Fair: Average rating is from 2 to less than 3
Failure: Average rating is less than 2

(2) Transparency

Each of the hair oils obtained in Examples and Comparative Examples was evaluated on transparency by visual observation according to criteria as follows:

Excellent: Transparent
Good: Semitransparent
Fair: Cloudy
Failure: Separated into two phases

[Table 1]

| | | Examples | | | | | | | | |
|---|---|---|---|---|---|---|---|---|---|---|
| | | 1 | 2 | 3 | 4 | 5 | 6 | 7 | 8 | 9 |
| Gel-forming agent | Lecithin | 5 | 5 | 5 | 5 | 5 | 5 | 5 | 5 | 5 |
| | Tetraglycerol | 1.8 | | | | | | | | |
| | Decaglycerol | | 2 | | | | | | | 0.25 |
| | Polyglycerol-20 | | | 2 | 2 | | | | 1 | |
| | Polyglycerol-20 monolaurate | | | | | 5 | | | | |
| | Polyglycerol-20 monomyristate | | | | | | 5 | | | |
| | Polyglycerol-40 monomyristate | | | | | | | 5 | | |
| Oil-phase component | Cetyl octanoate | 31 | 31 | 31 | 29.3 | 30 | 30 | 30 | | |
| | Sunflower oil | 31.1 | 31 | 31 | 29.3 | 30 | 30 | 30 | | |
| | Decamethylpentasiloxane | | | | 5 | | | | | |
| | Isododecane | 31.1 | 31 | 31 | 29.4 | 30 | 30 | 30 | | |
| | Liquid paraffin | | | | | | | | 94 | 94.8 |
| Evacuation | Thickness | Good | Good | Good | Good | Good | Good | Good | Good | Good |
| | Transparency | Excellent | Excellent | Excellent | Excellent | Good | Good | Good | Excellent | Excellent |
| | Smoothness | Good | Good | Good | Good | Good | Good | Good | Good | Good |

[Table 2]

| | | Comparative Examples | | | | | |
|---|---|---|---|---|---|---|---|
| | | 1 | 2 | 3 | 4 | 5 | 6 |
| Gel-forming agent | Lecithin | 5 | 5 | 5 | 5 | 5 | 5 |
| | Decaglycerol | | 10 | | | | 10 |
| | Polyglycerol-20 | | | 10 | | 10 | |
| | Polyglycerol-20 monolaurate | | | | 10 | | |
| Oil-phase component | Cetyl octanoate | 31.6 | 28.3 | 28.3 | 28.3 | | |
| | Sunflower oil | 31.7 | 28.3 | 28.3 | 28.3 | | |
| | Isododecane | 31.7 | 28.4 | 28.4 | 28.4 | | |
| | Liquid paraffin | | | | | 85 | 85 |
| Evaluation | Thickness | Failure | Failure | Failure | Failure | Failure | Failure |
| | Transparency | Failure | Failure | Failure | Failure | Failure | Failure |
| | Smoothness | Failure | Failure | Failure | Failure | Failure | Failure |

Industrial Applicability

The hair oils obtained according to the present invention spread smoothly during use, give a satisfactorily soft and light finish, highly effectively impart a moist feel, easiness of styling, and a glossy feel to the hair, and are very useful.

## Claims

1. A hair oil comprising an oil-based gel-like composition, the composition comprising a gel-forming agent; and an oil-phase component,
wherein:

    the gel-forming agent comprises: a lecithin; and at least one selected from the group consisting of a polyglycerol and a polyglycerol fatty acid ester in a total amount of from 5 to 100 percent by weight per 100 percent by weight of the lecithin;
    the polyglycerol has a degree of polymerization of from 2 to 20; and
    the polyglycerol fatty acid ester comprises a fatty acid residue having 14 or less carbon atoms, has an HLB of 15 or more as calculated based on an organic conceptual diagram, and has a degree of polymerization of from 8 to 40.

2. The hair oil according to claim 1, wherein the oil-based gel-like composition comprises 1 to 30 percent by weight of the gel-forming agent; and 70 to 99 percent by weight of the oil-phase component.

**INTERNATIONAL SEARCH REPORT**

International application No.
PCT/JP2012/070173

A. CLASSIFICATION OF SUBJECT MATTER
*A61K8/55*(2006.01)i, *A61K8/34*(2006.01)i, *A61K8/37*(2006.01)i, *A61K8/86*(2006.01)i, *A61K8/92*(2006.01)i, *A61Q5/06*(2006.01)i

According to International Patent Classification (IPC) or to both national classification and IPC

B. FIELDS SEARCHED

Minimum documentation searched (classification system followed by classification symbols)
A61K8/55, A61K8/34, A61K8/37, A61K8/86, A61K8/92, A61Q5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-63280 A (Cedarcos Japan Co., Ltd.), 21 March 2008 (21.03.2008), entire text (Family: none) | 1-2 |
| A | JP 2007-314442 A (Shiseido Co., Ltd.), 06 December 2007 (06.12.2007), entire text (Family: none) | 1-2 |
| A | JP 7-187947 A (The Nisshin Oil Mills, Ltd.), 25 July 1995 (25.07.1995), entire text (Family: none) | 1-2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 October, 2012 (29.10.12) | 06 November, 2012 (06.11.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/070173

C (Continuation). DOCUMENTS CONSIDERED TO BE RELEVANT

| Category* | Citation of document, with indication, where appropriate, of the relevant passages | Relevant to claim No. |
|---|---|---|
| A | WO 2011/052674 A1 (The Nisshin Oillio Group, Ltd.), 05 May 2011 (05.05.2011), entire text & CN 102481243 A | 1-2 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S63183517 A
- JP H01175923 A


### Non-patent literature cited in the description


- **TAKEO MITSUI.** *Shin Keshohin-gaku,* 12 January 1993, 436-439